# EUROPÄISCHE PATENTSCHRIFT

(11) **EP 1 172 066 B1**
(45) Veröffentlichungstag und Bekanntmachung des Hinweises auf die Patenterteilung: **02.11.2005**
(21) Anmeldenummer: 01116473.8
(22) Anmeldetag: 07.07.2001
(51) Int. Cl.: A61B 5/15

(54) **Abdichtung Kolbenstange für Spritze**
Syringe plunger rod
Tige de piston de séringue

(30) Priorität: 12.07.2000 DE 10033882
(43) Veröffentlichungstag der Anmeldung: 16.01.2002
(73) Patentinhaber: KABE-Labortechnik GmbH, 51588 Nümbrecht-Elsenroth (DE)
(72) Erfinder: Kolpe, Dieter, 51674 Wiehl (DE)
(74) Vertreter: Christophersen & Partner

(56) Entgegenhaltungen:
- DE-A- 2 456 561
- DE-A- 3 026 281
- DE-B- 1 184 042
- US-A- 3 937 211

## Beschreibung

Die Erfindung betrifft eine Blutentnahmevorrichtung mit einem zylindrischen Entnahmeröhrchen und mit einer Kolbenstange, die an ihrem einen Ende mit einem in dem Entnahmeröhrchen bewegbar geführten Kolben verbunden ist und an ihrem zweiten Ende ein Zugelement aufweist, wobei das Entnahmeröhrchen an einer Stirnseite eine Öffnung aufweist, durch die die Kolbenstange hindurchgeführt ist.

Aus dem Stand der Technik sind Blutentnahmevorrichtungen bekannt, die ein an einen Nadelhalter mit Kanüle ansetzbares Entnahmeröhrchen aufweisen, dessen vorderes Ende durch eine mit einem Stopfen versehene Kappe verschlossen ist. Die Blutentnahmevorrichtungen werden herstellerseitig keimfrei verpackt. Um die Blutentnahmevorrichtungen vor der Verwendung möglichst keimfrei zu halten und entnommenes Blut nicht durch Keime zu verunreinigen, werden diese Verpackungen erst kurz vor Gebrauch geöffnet. Bei einer einmal geöffneten Verpackung ist die Vorrichtung sofort zu verwenden, um eine Kontamination mit Keimen soweit als möglich zu unterbinden.

Aus Dokument DE 1 184 042 ist eine Blutentnahmevorrichtung mit den Merkmalen des Oberbegriffs bekannt.

Aufbauend auf dem bekannten Stand der Technik liegt der Erfindung die **Aufgabe** zugrunde, eine gattungsgemäße Vorrichtung zur Blutentnahme derart weiterzuentwickeln, daß eine Kontamination des inneren Entnahmeröhrchens der Blutentnahmevorrichtung nach Öffnen der Verpackung durch Keime weitgehend vermieden wird, wobei die Blutentnahmevorrichtung kompakt gestaltet und preisgünstig herstellbar sein soll.

Die Aufgabe wird dadurch **gelöst**, daß das Entnahmeröhrchen im Bereich der Öffnung mit einem umlaufend ausgebildeten Dichtelement versehen ist, welches zumindest in der am meisten eingeschobenen axialen Stellung der Kolbenstange eine über den gesamten Umfang der Öffnung wirksame Dichtung bildet.

Der Raum innerhalb des Blutentnahmeröhrchens wird auf diese Weise auch nach dem Öffnen der sterilen Verpackung durch die Dichtung gegen eine Kontamination mit Keimen geschützt werden. Gerade im Operationsbereich eines Krankenhauses oder im Rahmen einer Notfallversorgung kann durch die erfindungsgemäße Dichtwirkung eine zusätzliche Sicherheit gegen eine Kontamination der Blutentnahmevorrichtung mit Keimen gewährleistet werden. Daneben steht vorteilhaft nahezu das gesamte Volumen des Entnahmeröhrchens zur Aufnahme der Blutprobe zur Verfügung. Das Blutentnahmeröhrchen baut daher kompakt und kann mit üblichen Kunststoffspritzverfahren kostengünstig produziert werden.

Ferner wird vorgeschlagen, daß das Dichtelement zugleich eine Lagerung für die Kolbenstange bildet. Die Führung kann eine Bedienung bei einer Blutentnahme erleichtern und daneben bei Kolbenstangen mit einer Sollbruchstelle am Kolben eine definierte axiale Bruchstellung gewährleisten, wenn das Blutentnahmeröhrchen bestimmungsgemäß gefüllt ist. Darüber hinaus kann hierdurch ein Füllvolumen vorgegeben werden.

Das Dichtelement ist durch eine Ausformung der Stirnseite des Entnahmeröhrchens als Dichtlippe gebildet. Die Dichtlippe kann neben der Lagerung der Kolbenstange zusammen mit der Kolbenstange eine Dichtung bilden, durch die eine Kontamination des Innenraumes des Blutentnahmeröhrchens vermieden wird. Zudem können Herstellkosten reduziert werden, indem das Dichtelement einstückig mit dem Blutentnahmeröhrchen ausgeführt wird und somit Bauteile und Aufwand für deren Montage entfällt.

In einer Ausgestaltung wird vorgeschlagen, daß die Kolbenstange einen umlaufenden Kragen aufweist, der in der Dichtstellung mit der Dichtlippe zusammenwirkt. In der am meisten eingeschobenen axialen Stellung kann eine besonders wirksame Dichtwirkung erreicht werden, wodurch die Möglichkeit einer Kontamination mit Keimen bei geöffneter Sterilverpackung weiter reduziert werden kann.

Vorteilhaft weist der Kragen in Richtung Kolben eine umlaufende Ausnehmung auf, in die die Dichtlippe in der Dichtstellung eingreift. Dadurch kann die Dichtwirkung weiter verbessert werden, beispielsweise durch Aufbau als Labyrinthdichtung.

Es wird ferner vorgeschlagen, daß der Kragen als Konus ausgebildet ist, der in der Dichtstellung in eine entsprechend konisch ausgeformte Dichtlippe greift. Die Form des Konus führt zu einer großen Dichtfläche und demzufolge zu einer guten Dichtwirkung. Um zusätzlich zu einer großen Dichtfläche einen Ausgleich von Toleranzen zu ermöglichen wird vorgeschlagen, daß der Kragen der Kolbenstange als Kugelkalotte ausgeformt ist, die in der Dichtstellung mit der Dichtlippe zusammenwirkt.

Um bei kleinen axialen Auslenkungen aus der Dichtstellung keinen Verlust der Dichtwirkung zu erreichen, wird vorgeschlagen, daß die Kolbenstange axial anschließend am Kragen in Richtung Kolben eine zylindrische Oberfläche aufweist, auf der die Dichtlippe anliegt. Beispielsweise kann die Blutentnahmevorrichtung bei geöffneter Sterilverpackung weiterhin keimfrei bleiben, auch wenn versehentlich die Kolbenstange axial aus ihrer Dichtstellung bewegt worden ist.

Eine weitere Steigerung der Dichtwirkung kann erreicht werden, indem die Kolbenstange axial zwischen dem Kragen und dem Kolben eine umlaufende, wulstförmige Ausformung aufweist. Daneben kann die Ausformung auch eine Rastfunktion darstellen, durch die ein versehentliches Bewegen der Kolbenstange aus ihrer Dichtstellung behindert wird, beispielsweise bei einer Entnahme aus der Sterilverpackung.

Weitere Vorteile und Merkmale sind der folgenden Beschreibung von Ausführungsbeispielen zu entnehmen. Dabei sind gleiche Bauteile einheitlich mit gleichen Bezugszeichen versehen.

Es zeigen:
- Fig. 1: eine Blutentnahmevorrichtung in Gesamtansicht,
- Fig. 2: einen vergrößerten Ausschnitt des Dichtelements aus Fig. 1,
- Fig. 3: einen vergrößerten Ausschnitt eines weiteren Dichtelements,
- Fig. 4: einen Ausschnitt des Dichtelements, das in eine Ausnehmung eines Kragens einer Kolbenstange eingreift,
- Fig. 5: einen Ausschnitt eines konisch geformten Dichtelements,
- Fig. 6: einen Ausschnitt ein an einem zylindrischen Bereich der Kolbenstange anliegendes Dichtelement,
- Fig. 7: einen Ausschnitt einer Blutentnahmevorrichtung mit einer über den gesamten Stellweg zylindrisch geformten Kolbenstange,
- Fig. 8: einen Ausschnitt im Bereich des Dichtelements aus Fig. 2 mit einer zusätzlich zum Kragen wulstförmigen Ausformung der Kolbenstange,
- Fig. 9: die Gesamtansicht der Blutentnahmevorrichtung nach Fig. 7,
- Fig. 10: einen Ausschnitt einen Ausschnitt einer Blutentnahmevorrichtung nach Fig. 8 mit einem aus dem Ende der Entnahmeröhre gebildeten Dichtelements,
- Fig. 11: einen Ausschnitt einer Blutentnahmevorrichtung mit einem aus dem Mantel der Entnahmeröhre und dem Kragen gebildeten Dichtelement,
- Fig. 12: einen Ausschnitt einer Blutentnahmevorrichtung mit einem aus dem Kragen und der äußeren Oberfläche der Entnahmeröhre gebildeten Dichtelement,
- Fig. 13: eine Blutentnahmevorrichtung in Gesamtansicht mit einem aus dem Kragen und der inneren Oberfläche der Entnahmeröhre gebildeten Dichtelement,
- Fig. 14: das Dichtelement aus Fig. 13 und
- Fig. 15: eine Gesamtansicht einer Blutentnahmevorrichtung nach Fig. 10.

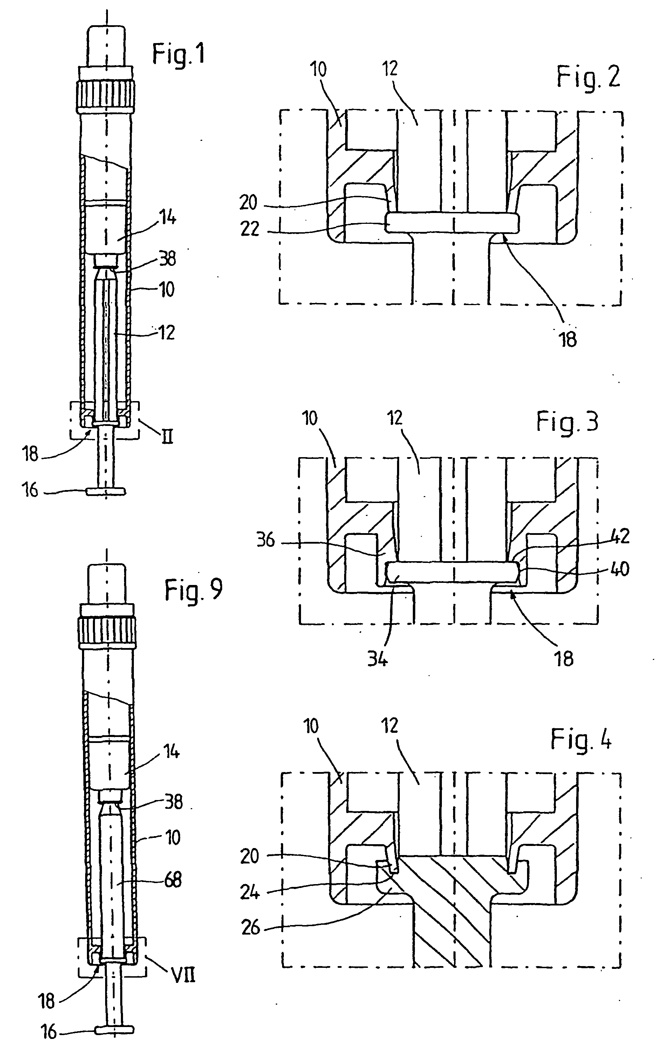

Fig. 1 zeigt eine Blutentnahmevorrichtung mit einem zylindrischen Entnahmeröhrchen 10 und mit einer Kolbenstange 12, die an ihrem einen Ende mit einem in dem Entnahmeröhrchen 10 bewegbar geführten Kolben 14 verbunden ist und an ihrem zweiten Ende ein Zugelement 16 in Gestalt einer Erweiterung der Kolbenstange aufweist. Eine Verbindungsstelle 38, über die die Kolbenstange 12 mit dem Kolben 14 verbunden ist, ist als Schwachstelle ausgeführt, an der in der am meisten axial herausgezogenen Stellung die Kolbenstange 12 abbrechbar ist. Das Entnahmeröhrchen 10 weist zudem an einer Stirnseite eine Öffnung 18 auf, durch die die Kolbenstange 12 hindurchgeführt ist.

Erfindungsgemäß ist das Entnahmeröhrchen 10 im Bereich der Öffnung 18 mit einem umlaufend ausgebildeten ringförmigen Dichtelement 20 versehen, welches in der am meisten eingeschobenen axialen Stellung der Kolbenstange 12 eine über den gesamten Kreisumfang der Öffnung 18 wirksame Dichtung bildet (Fig. 2). Das Dichtelement 20 bildet zugleich eine Lagerung für die Kolbenstange 12 und ist durch eine Ausformung der Stirnseite des Entnahmeröhrchens 10 als Dichtlippe ausgebildet. Die Kolbenstange 12 weist einen umlaufenden Kragen 22 auf, der in der Dichtstellung mit der Dichtlippe 20 zusammenwirkt. Grundsätzlich ist es sinnvoll, daß die Kolbenstange 12 zunächst in ihrer am meisten eingeschobenen Stellung gehalten ist, damit beim Auspacken der Blutentnahmevorrichtung aus einer nicht näher dargestellten Verpackung die Dichtwirkung des Dichtelements 20 nicht durch eine axiale Bewegung der Kolbenstange 12 aufgehoben wird, wodurch eine Kontamination mit Keimen ermöglicht werden kann. Daher unterstützt die durch das Aufliegen der Dichtlippe 20 auf der Kolbenstange 12 erzeugte Reibungskraft das Verharren der Kolbenstange 12 in ihrer am meisten eingeschobenen axialen Stellung.

In Fig. 3 ist eine weitere erfindungsgemäße Ausgestaltung der in Fig. 1 dargestellten Blutentnahmevorrichtung gezeigt, bei der ein Kragen 34 in eine Dichtlippe 36 vollständig bis zu einem von der Dichtlippe 36 gebildeten Anschlag 42 versenkbar ist. Ein Dichtbereich 40 wird durch Zusammenwirken der Dichtlippe 36 mit der radial äußersten Ausdehnung des Kragens 34 gebildet, wodurch eine Unterstützung der Reibungskraft erreichbar ist, die die Kolbenstange 12 in ihrer am meisten eingeschobenen axialen Stellung hält.

Fig. 4 zeigt einen Ausschnitt einer weiteren Ausgestaltung einer Blutentnahmevorrichtung mit einem Kragen 26, der auf der dem Dichtelement 20 zugewandten Seite eine umlaufende Ausnehmung 24 aufweist, in die die Dichtlippe 20 in der Dichtstellung eingreift. Auch in diesem Beispiel ist über eine zusätzliche Reibungskraft zwischen der Dichtlippe 20 und dem Kragen 26 die Fixierung der Kolbenstange 12 in ihrer am meisten eingeschobenen axialen Stellung unterstützt.

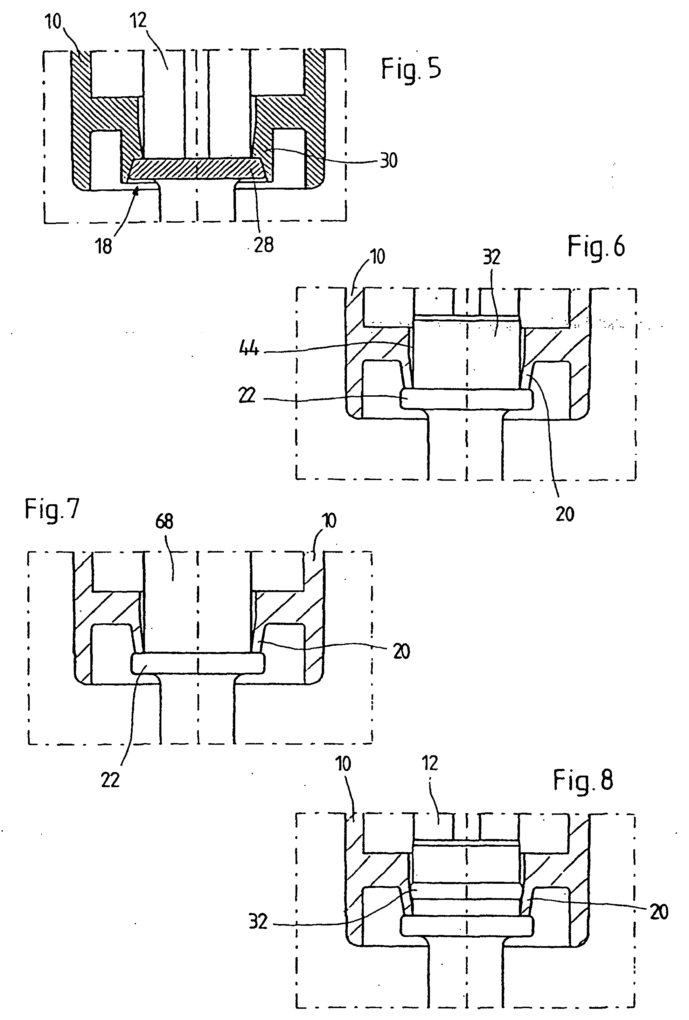

Um eine möglichst große Dichtfläche zu erreichen, ist in dem in Fig. 5 dargestellten Ausführungsbeispiel der Kragen 28 als Konus ausgeführt, der in der Dichtstellung in einem durch die Dichtlippe 30 gebildeten Gegenkonus anliegt. Anstelle eines Konus kann auch eine Kugelkalotte verwendet werden, wobei die Dichtlippe in der Dichtstellung auf der Kugelkalotte aufliegt. Diese Anordnung kann beispielsweise die Dichtwirkung auch bei axialen Toleranzen der Kolbenstange und / oder des Entnahmeröhrchens ermöglichen.

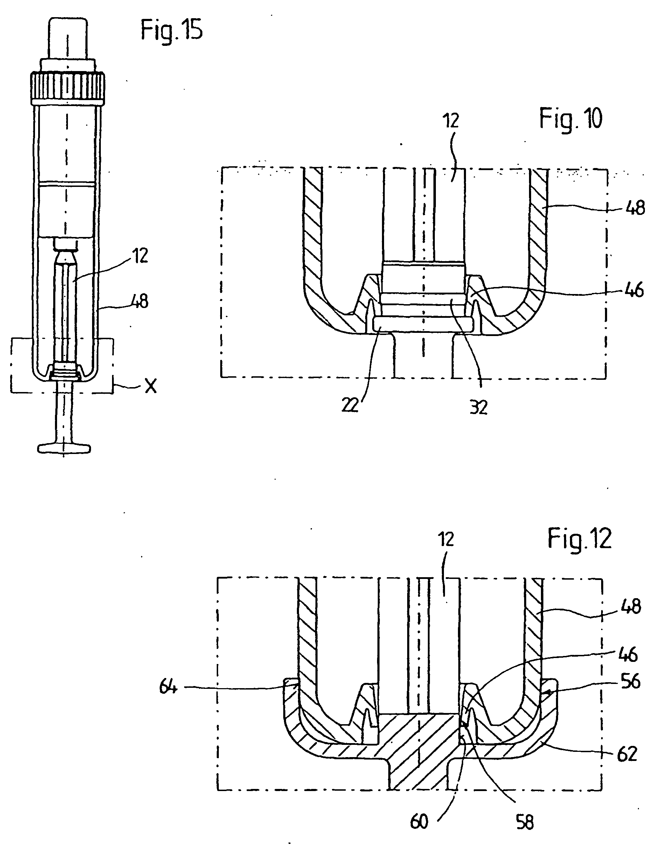

In einer weiteren erfindungsgemäßen Ausgestaltung in Fig. 6 weist die Kolbenstange 12 axial anschließend am Kragen 22 im Entnahmeröhchen 10 eine zylindrische Oberfläche 44 auf, auf der die Dichtlippe 20 anliegt. Diese Anordnung ermöglicht die Dichtwirkung auch bei geringfügig aus der Dichtstellung bewegter Kolbenstange 12. Fig. 7 und 9 zeigen eine weitere Ausgestaltung, bei der die Kolbenstange 68 vom Kragen 22 bis zur Verbindungsstelle 38 eine zylindrische Oberfläche aufweist.

Um ein besonders wirkungsvolles Dichtelement 20 zu erhalten und gleichzeitig eine Rastwirkung in der Dichtstellung zu erreichen, weist das in Fig. 8 gezeigte Ausführungsbeispiel einen Dichtwulst 32 am zylindrischen Abschnitt der Kolbenstange 12 auf. Dabei ist die Rastwirkung für einen bestimmungsgemäßen Gebrauch durch Form des Wulstes 32 und der über die Dichtlippe 20 auf den Wulst 32 wirkenden Kraft ergonomisch ausgelegt, und zwar so, daß eine Rastwirkung erreicht wird, aber die aufzubringende axiale Kraft eine Bedienung der Blutentnahmevorrichtung nicht wesentlich erschwert. Alternativ zeigt Fig. 10 ein Entnahmeröhrchen 48 mit einem als Dichtlippe 46 ausgebildeten Endbereich, in dem die Kolbenstange 12 mit dem Wulst 32 aus Fig. 8 eingesetzt ist. Die Gesamtansicht ist in Fig. 15 dargestellt.

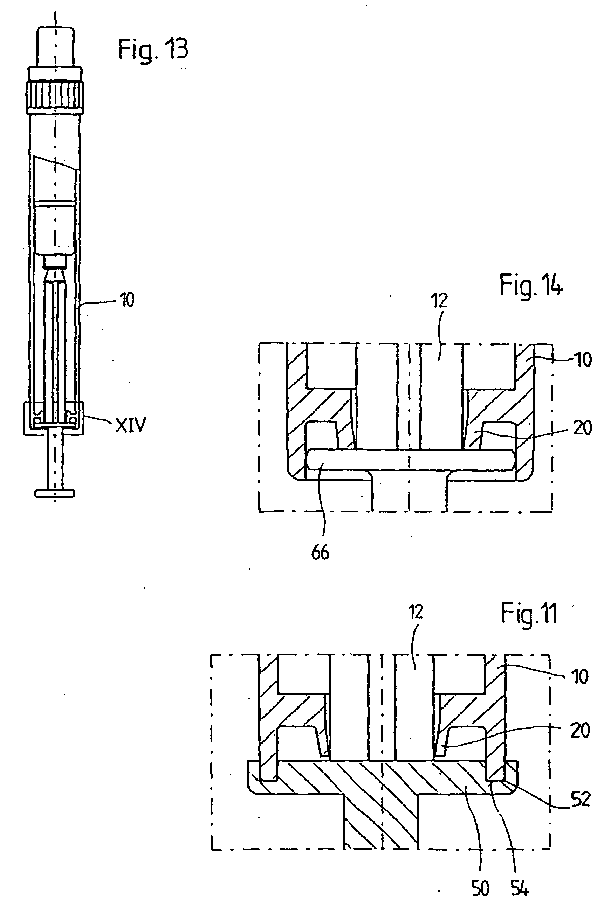

Fig. 11 zeigt einen Ausschnitt einer Blutentnahmevorrichtung mit einem Entnahmeröhrchen 10 gemäß Fig. 2, bei der die Kolbenstange 12 einen Kragen 50 mit einer Ausnehmung 52 zur dichtenden Aufnahme des stirnseitigen Rands 54 des Entnahmeröhrchens 10 aufweist. In der Dichtstellung bildet der Rand 54 der Wand des Entnahmeröhrchens 10 mit dem Kragen 50 ein Dichtelement. Die Lippe 20 dient als Führung für die Kolbenstange 12.

Aufbauend auf Fig. 10 zeigt Fig. 12 eine weitere Ausgestaltung mit zwei Dichtbereichen 56, 58. Das Entnahmeröhrchen 48 weist im Endbereich eine Dichtlippe 46 auf, die in der Dichtstellung auf einer Dichtfläche 60 der Kolbenstange 12 aufliegt und einen ersten Dichtbereich 58 bildet. Ein axial sich anschließender Kragen 62 der Kolbenstange 12 außerhalb des Entnahmeröhrchens 10 ist derart ausgebildet, daß er radial außen in Richtung Entnahmeröhrchen 10 umgebogen ist und mit einer äußeren Oberfläche 64 des Entnahmeröhrchens 48 in der Dichtstellung den Dichtbereich 56 bildet. Diese Kette aus zwei Dichtbereichen 56, 58 bietet hohe Sicherheit bezüglich einer Kontamination mit Keimen, insbesondere auch wenn beispielsweise beim Transport eine Verpackung beschädigt worden ist und bei Vorrichtungen gemäß dem Stand der Technik üblicherweise eine Kontamination anzunehmen ist. Fig. 13 zeigt die Gesamtansicht einer Blutentnahmevorrichtung mit einem Entnahmeröhrchen 10 ähnlich wie in Fig. 2. Abweichend zu dem in Fig. 2 dargestellten Ausführungsbeispiel weist die Blutentnahmevorrichtung einen Kragen 66 (Fig. 14) der Kolbenstange 12 mit einer abgerundeten umlaufenden Kante auf, die in der Dichtstellung mit einer Innenwandung des Entnahmeröhrchens 10 eine weitere Dichtung bildet. Der Aufbau und die Funktion des inneren Dichtelements entspricht dem der Fig. 2.

### Bezugszeichenliste

- 10: Entnahmeröhrchen
- 12: Kolbenstange
- 14: Kolben
- 16: Zugelement
- 18: Öffnung
- 20: Dichtelement
- 22: Kragen
- 24: Ausnehmung
- 26: Kragen
- 28: Kragen
- 30: Dichtlippe
- 32: Ausformung
- 34: Kragen
- 36: Dichtlippe
- 38: Verbindungsstelle
- 40: Dichtbereich
- 42: Anschlag
- 44: Oberfläche
- 46: Dichtlippe
- 48: Entnahmeröhrchen
- 50: Kragen
- 52: Ausnehmung
- 54: Rand
- 56: Dichtbereich
- 58: Dichtbereich
- 60: Dichtfläche
- 62: Kragen
- 64: Oberfläche
- 66: Kragen
- 68: Kolbenstange

## Patentansprüche

1. Blutentnahmevorrichtung mit einem zylindrischen Entnahmeröhrchen (10, 48) und mit einer Kolbenstange (12, 68), die an ihrem einen Ende mit einem in dem Entnahmeröhrchen (10, 48) bewegbar geführten Kolben (14) verbunden ist und an ihrem zweiten Ende ein Zugelement (16) aufweist, wobei das Entnahmeröhrchen (10, 48) an einer Stirnseite eine Öffnung (18) aufweist, die mit einem umlaufend ausgebildeten Dichtelement (20, 36) versehen ist, und durch die die Kolbenstange (12, 68) hindurchgeführt ist, und wobei das Dichtelement (20, 36) zumindest in der am meisten eingeschobenen axialen Stellung der Kolbenstange (12, 68) eine über den gesamten Umfang der Öffnung (18) wirksame Dichtung bildet,
**dadurch gekennzeichnet,**
**daß** das Dichtelement (20, 36) durch eine einstückige Ausformung der Stirnseite des Entnahmeröhrchens (10, 48) als Dichtlippe gebildet ist.

2. Blutentnahmevorrichtung nach Anspruch 1, **dadurch gekennzeichnet, daß** das Dichtelement (20, 36) zugleich eine Lagerung für die Kolbenstange (12, 68) bildet.

3. Blutentnahmevorrichtung nach einem der vorhergehenden Ansprüche, **dadurch gekennzeichnet, daß** die Kolbenstange (12, 68) einen umlaufenden Kragen (22, 34, 50, 62) aufweist, der in der Dichtstellung mit der Dichtlippe (20, 36, 46) zusammenwirkt.

4. Blutentnahmevorrichtung nach Anspruch 3, **dadurch gekennzeichnet, daß** der Kragen (26) in Richtung Kolben (14) eine umlaufende Ausnehmung (24) aufweist, in die die Dichtlippe (20) in der Dichtstellung eingreift.

5. Blutentnahmevorrichtung nach Anspruch 3 oder 4, **dadurch gekennzeichnet, daß** der Kragen (50) in Richtung Kolben (14) eine umlaufende Ausnehmung (52) aufweist, in die ein Stirnbereich (54) des Entnahmeröhrchens (10) in der Dichtstellung eingreift.

6. Blutentnahmevorrichtung nach einem der vorhergehenden Ansprüche, **dadurch gekennzeichnet, daß** der Kragen (34) als Konus ausgebildet ist, der in der Dichtstellung in eine entsprechend konisch ausgeformte Dichtlippe (36) greift.

7. Blutentnahmevorrichtung nach einem der Ansprüche 1 bis 2, **dadurch gekennzeichnet, daß** der Kragen der Kolbenstange (12) als Kugelkalotte ausgeformt ist, die in der Dichtstellung mit der Dichtlippe zusammenwirkt.

8. Blutentnahmevorrichtung nach einem der vorhergehenden Ansprüche, **dadurch gekennzeichnet, daß** die Kolbenstange (12, 68) axial anschließend am Kragen (22, 62) in Richtung Kolben (14) eine zylindrische Oberfläche (44, 60) aufweist, auf der die Dichtlippe (20, 46) anliegt.

9. Blutentnahmevorrichtung nach einem der vorhergehenden Ansprüche, **dadurch gekennzeichnet, daß** die Kolbenstange (12) axial zwischen dem Kragen (22) und dem Kolben (14) eine umlaufende, wulstförmige Ausformung (32) aufweist.

## Claims

1. Device for taking a blood sample, comprising a cylindrical sample tube (10, 48) and a piston rod (12, 68) which at its first end is connected to a piston (14) which is guided movably in the sample tube (10, 48) and at its second end has a pulling element (16), wherein the sample tube (10, 48) has at its end an opening (18) which is provided with a peripheral sealing element (20, 36) and through which the piston rod (12, 68) is passed, and wherein the sealing element (20, 36) forms a seal which is effective over the entire circumference of the opening (18) at least in the maximally inserted axial position of the piston rod (12, 68), **characterized in that** the sealing element (20, 36) is formed as a sealing lip by an integral shaping of the end of the sample tube (10, 48).

2. Device for taking a blood sample according to Claim 1, **characterized in that** the sealing element (20, 36) at the same time forms a bearing for the piston rod (12, 68).

3. Device for taking a blood sample according to either of the preceding claims, **characterized in that** the piston rod (12, 68) has a peripheral collar (22, 34, 50, 62) which in the sealed position cooperates with the sealing lip (20, 36, 46).

4. Device for taking a blood sample according to Claim 3, **characterized in that** the collar (26) has, in the direction of the piston (14), a peripheral recess (24) in which the sealing lip (20) engages in the sealed position.

5. Device for taking a blood sample according to Claim 3 or 4, **characterized in that** the collar (50) has, in the direction of the piston (14), a peripheral recess (52) in which an end region (54) of the sample tube (10) engages in the sealed position.

6. Device for taking a blood sample according to any of the preceding claims, **characterized in that** the collar (34) is designed as a cone which in the sealed position engages in a corresponding conical sealing lip (36).

7. Device for taking a blood sample according to either of Claims 1 and 2, **characterized in that** the collar of the piston rod (12) is shaped as a spherical cap which in the sealed position cooperates with the sealing lip.

8. Device for taking a blood sample according to any of the preceding claims, **characterized in that** the piston rod (12, 68) has, axially adjoining the collar (22, 62) in the direction of the piston (14), a cylindrical surface (44, 60) against which the sealing lip (20, 46) bears.

9. Device for taking a blood sample according to any of the preceding claims, **characterized in that** the piston rod (12) has, axially between the collar (22) and the piston (14), a peripheral bead-like formation (32).

## Revendications

1. Dispositif de prélèvement de sang avec un tube de prélèvement cylindrique (10, 48) et avec une tige de piston (12, 68) qui est reliée à l'une de ses extrémités avec un piston (14) guidé de manière mobile dans le tube de prélèvement (10, 48) et qui comprend à sa deuxième extrémité un élément de tirage (16), le tube de prélèvement (10, 48) comprenant sur un côté frontal une ouverture (18) qui est munie d'un élément d'étanchéité (20, 36) configuré de manière périphérique et à travers laquelle passe la tige de piston (12, 68) et l'élément d'étanchéité (20, 36) formant, au moins dans la position la plus introduite dans le sens axial de la tige de piston (12, 68), une étanchéité agissant sur la totalité du pourtour de l'ouverture (18), **caractérisé en ce que** l'élément d'étanchéité (20, 36) est constitué par une mise en forme en une seule pièce du côté frontal du tube de prélèvement cylindrique (10, 48) en tant que lèvre d'étanchéité.

2. Dispositif de prélèvement de sang selon la revendication 1, **caractérisé en ce que** l'élément d'étanchéité (20, 36) forme en même temps un logement pour la tige de piston (12, 68).

3. Dispositif de prélèvement de sang selon l'une quelconque des revendications précédentes, **caractérisé en ce que** la tige de piston (12, 68) comprend un collet circulaire (22, 34, 50, 62) qui coopère avec la lèvre d'étanchéité (20, 36, 46) en position d'étanchéité.

4. Dispositif de prélèvement de sang selon la revendication 3, **caractérisé en ce que** le collet (26) comprend en direction du piston (14) un évidemment circulaire (24) dans lequel pénètre la lèvre d'étanchéité (20) en position d'étanchéité.

5. Dispositif de prélèvement de sang selon la revendication 3 ou 4, **caractérisé en ce que** le collet (50) comprend en direction du piston (14) un évidemment circulaire (52) dans lequel pénètre une zone frontale (54) du tube de prélèvement cylindrique (10) en position d'étanchéité.

6. Dispositif de prélèvement de sang selon l'une quelconque des revendications précédentes, **caractérisé en ce que** le collet (34) est configuré comme cône qui pénètre dans une lèvre d'étanchéité (36) formée de manière conique correspondante en position d'étanchéité.

7. Dispositif de prélèvement de sang selon l'une quelconque des revendications 1 à 2, **caractérisé en ce que** le collet de la tige de piston (12) est configuré comme calotte sphérique qui agit de concert avec la lèvre d'étanchéité en position d'étanchéité.

8. Dispositif de prélèvement de sang selon l'une quelconque des revendications précédentes, **caractérisé en ce que** la tige de piston (12, 68) comprend dans le sens axial à la suite du collet (22, 62) en direction du piston (14) une surface cylindrique (44, 60) sur laquelle s'appuie la lèvre d'étanchéité (20, 46).

9. Dispositif de prélèvement de sang selon l'une quelconque des revendications précédentes, **caractérisé en ce que** la tige de piston (12) comprend dans le sens axial entre le collet (22) et le piston (14) une conformation circulaire en forme de boudin (32).
